# EUROPEAN PATENT SPECIFICATION

(11) **EP 2 178 519 B1**
(45) Date of publication and mention of the grant of the patent: **25.04.2012**
(21) Application number: 08762998.6
(22) Date of filing: 30.06.2008
(51) Int. Cl.: A61K 31/00, A61K 31/42, A61K 31/415, A61K 31/4706, A61K 31/635, A61K 9/51, A61K 47/38

(54) **Nanoparticles comprising a non-ionizable polymer and an anionic cellulosic polymer**
Nanopartikel mit einem nicht ionisierbaren Polymer und einem anionischen Zellulosepolymer
Nanoparticules comprenant un polymère non ionisable et un polymère cellulosique anionique

(30) Priority: 13.07.2007 US 949522 P
(43) Date of publication of application: 28.04.2010
(73) Proprietor: Bend Research, Inc., Bend, OR 97701-8599 (US)
(72) Inventor: BLOOM, Corey, Jay, Bend, Oregon 97701 (US); CREW, Marshall, David, Bend, Oregon 97701 (US); MILLER, Warren, Kenyon, Bend, Oregon 97701 (US); SMITHEY, Daniel, Tod, Bend, Oregon 97701 (US)
(74) Representative: Beckmann, Claus
(86) International application number: PCT/IB2008/001776
(87) International publication number: WO 2009/010837

(56) References cited:
- WO-A-99/33558
- PINON-SEGUNDO E ET AL: "Preparation and characterization of triclosan nanoparticles for periodontal treatment" INTERNATIONAL JOURNAL OF PHARMACEUTICS, ELSEVIER BV, NL, vol. 294, no. 1-2, 27 April 2005 (2005-04-27), pages 217-232, XP004825476 ISSN: 0378-5173

## Description

### BACKGROUND OF THE INVENTION

The present invention relates to nanoparticles comprising a poorly water- soluble drug, a poorly aqueous soluble non-ionizable polymer and an anionic cellulosic polymer.

A variety of approaches have been taken to formulate drugs as nanoparticles. One approach is to decrease the size of a crystalline drug by grinding or milling the drug in the presence of a surface modifier. See, e.g., U.S. Patent No. 5,145,684. Another approach to forming nanoparticles is to precipitate the drug in the presence of a film forming material such as a polymer. See, e.g., U.S. Patent No. 5,118,528.

Nanoparticles have been formed from a variety of polymers. Bodmeier and Chen (J. Controlled Release, 12, (1990) 223-233) disclose forming nanoparticles with a non-ionizable polymer such as ethylcellulose or with a cationic polymer such as Eudragit RL or RS. Gumy, et al., US Patent Application 2044/0018236 A1 disclose nanoparticles comprising drugs having low water solubility and anionic polymers. The polymer is enteric, i.e., resistant to gastric juices and soluble in intestinal juices. Exemplary enteric polymers include Eudragit L and Eudragit S, polyvinyl acetate phthalate, hydroxypropylmethyl cellulose acetate succinate, hydroxypropylmethyl cellulose phthalate, cellulose acetate phthalate, and cellulose acetate trimellitate. Nanoparticles made with Eudragit L and Eudragit S are exemplified.

Pinon-Segundo E. et al. disclose triclosan - loaded nanoparticles prepared by a emulsification - diffusion process using poly(d,1 - lactide - co - glycolide) (PLGA), poly (d,1 - lactide) (PLA) or cellulose acetate phthalate (CAP). Nanoparticles are obtained with a diameter of less than 500 nm (Pinon-Segundo E. et al.: "Preparation and characterization of triclosan nanoparticles for periodontal treatment", International Journal of Pharmaceutics, vol. 294, no. 1-2, (2005), pages 217-232).

WO99/33558 discloses a method for producing an aqueous colloidal dispersion of nanoparticles, characterized in that it comprises:
a) the emulsification of an organic solvent containing a polymer in an aqueous solution of a stabilizing agent,
b) the distillation of the organic solvent from the oil - in - water emulsion formed in step a) to cause the formation of nanoparticles, in suspension in the aqueous phase.

In WO99/33558 the polymer is selected from polymers such as poly(D,L- lactic acid) (PLA), poly(ε - caprolactone) (PCL), cellulose acetate phthalate (CAP) and cellulose acetate trimellitate (CAT).

### BRIEF SUMMARY OF THE INVENTION

In a first aspect, a pharmaceutical composition comprising nanoparticles, said nanoparticles comprising:
(a) a drug having a solubility in water of less than 5mg/mL over the pH range of 6.5 to 7,5 at 25°C, at least 90 wt% of said drug being non-crystalline;
(b) a non-ionizable polymer having a solubility of less than 0.1 mg/mL at a concentration of 0,2 mg/mL in a phosphate buffered aqueous saline solution at pH 6.5 (i.e. a poorly aqueous soluble non-ionizable polymer); and
(c) an anionic cellulosic polymer;
   wherein said nanoparticles have an average size of less than 500 nm and said drug, said non-ionizable polymer, and said anionic cellulosic polymer collectively constitute at least 80 wt% of said nanoparticles.

In one embodiment, the poorly aqueous soluble non-ionizable polymer is selected from the group consisting of ethylcellulose, cellulose acetate, cellulose propionate, cellulose butyrate, and cellulose acetate butyrate.

In another embodiment, the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, hydroxypropyl methyl cellulose acetate phthalate, cellulose acetate trimellitate, hydroxypropyl methyl cellulose acetate trimellitate, ethyl cellulose succinate, ethyl cellulose phthalate, ethyl cellulose trimellitate, cellulose acetate succinate, and methylcellulose acetate succinate.

In yet another embodiment, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, and cellulose acetate succinate.

In still another embodiment, the non-ionizable polymer is ethylcellulose and the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, and cellulose acetate succinate.

Because the nanoparticles are formed from a blend of a poorly aqueous soluble non-ionizable polymer and an anionic cellulosic polymer, the stability of the non-crystalline drug and the suspension stability of the nanoparticle can be addressed independently, resulting in nanoparticles with improved performance and stability.

First, the poorly aqueous soluble non-ionizable polymer used in the nanoparticles stabilizes the poorly water soluble drug in the sense of reducing the rate of crystallization of the drug in the solid state and while in suspension. In addition, because the non-ionizable polymer is poorly aqueous soluble at physiological pH, the nanoparticles maintain the drug within a solid (or at least undissolved) polymer matrix when the nanoparticles are suspended in an aqueous solution, further preventing or reducing crystallization of the drug. It is well known that the non-crystalline form of a low-solubility drug provides a greater aqueous concentration of drug relative to the crystalline form of the drug when administered to an aqueous use environment. However, it is also well known that when the drug is not stabilized in the non-crystalline form, the drug rapidly converts to the crystalline form in the use environment. See, for example, Hancock and Parks (Pharmaceutical Research, Vol. 17, No. 4, 2000). Thus, the poorly aqueous soluble non-ionizable polymer is used to maintain the stability of the non-crystalline drug in the nanoparticle and while suspended in an aqueous solution, resulting in an enhanced concentration of free dissolved drug when the nanoparticle is administered to an aqueous use environment.

Second, the anionic cellulosic polymer provides a charge when the nanoparticles are suspended in an aqueous use environment, thus reducing or eliminating agglomeration of the nanoparticles. The anionic cellulosic polymer also results in improved re-suspendability of solid compositions containing nanoparticles relative to surfactant-based and neutral polymer-based stabilizers: solid compositions of the invention resuspend nanoparticles when administered to an aqueous solution.

The combination of the two different polymers-the non-ionizable polymer and the anionic cellulosic polymer-provides a synergistic effect resulting in improved stability and re-suspendability of the nanoparticles.

The foregoing and other objectives, features, and advantages of the invention will be more readily understood upon consideration of the following detailed description of the invention.

### BRIEF DESCRIPTION OF THE SEVERAL VIEWS OF THE DRAWINGS

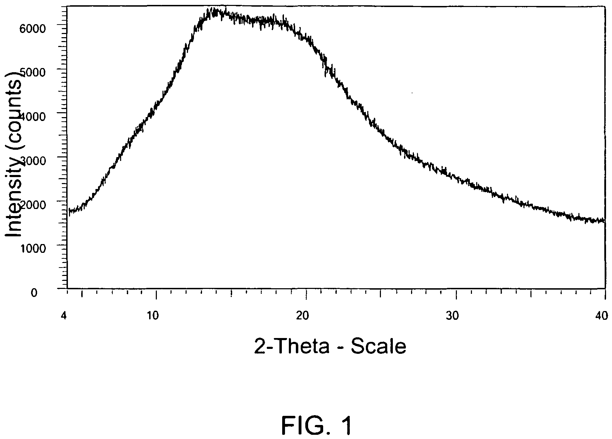

FIG. 1 is a powder X ray diffraction (PXRD) diffractogram of the lyophilized nanoparticles of Example 6.

### DETAILED DESCRIPTION OF THE INVENTION

The nanoparticles of the present invention comprise a poorly water soluble drug, a poorly aqueous soluble non-ionizable polymer, and an anionic cellulosic polymer. At least 90 wt% of the drug in the nanoparticle is non-crystalline. The nature of the nanoparticles, suitable anionic cellulosic polymers and drugs, and methods for making nanoparticles are described in detail below.

### Nanoparticles

The nanoparticles comprise the drug, the non-ionizable polymer and the anionic cellulosic polymer. By "nanoparticles" is meant a plurality of small particles in which the average size of the particles is less than about 500 nm. In suspension, by "average size" is meant the effective cumulant diameter as measured by dynamic light scattering (DLS), using for example, Brookhaven Instruments' 90Plus particle sizing instrument. By "size" is meant the diameter if the particles were spherical particles, or the maximum diameter for non-spherical particles. Preferably, the average size of the nanoparticles is less than 400 nm, more preferably less 300 nm, more preferably less than 200 nm, more preferably less than 150 nm, and most preferably less than 100 nm.

The width of the particle size distribution in suspension is given by the "polydispersity" of the particles, which is defined as the relative variance in the correlation decay rate distribution, as is known by one skilled in the art. See B.J. Fisken, "Revisiting the method of cumulants for the analysis of dynamic light-scattering data," Applied Optics, 40(24), 4087-4091 (2001) for a discussion of cumulant diameter and polydispersity. Preferably, the polydispersity of the nanoparticles is less than 0.5. More preferably, the polydispersity of the nanoparticles is less than about 0.3. In one embodiment, the average size of the nanoparticles is less than 500 nm with a polydispersity of 0.5 or less. In another embodiment, the average size of the nanoparticles is less than 300 nm with a polydispersity of 0.5 or less. In still another embodiment, the average size of the nanoparticles is less than 200 nm with a polydispersity of 0.5 or less. In yet another embodiment, the average size of the nanoparticles is less than 200 nm with a polydispersity of 0.3 or less.

At least 90 wt% of the drug in the nanoparticles is non-crystalline. The term "crystalline," as used herein, means a particular solid form of a compound that exhibits long-range order in three dimensions. "Non-crystalline" refers to material that does not have long-range three-dimensional order, and is intended to include not only material which has essentially no order, but also material which may have some small degree of order, but the order is in less than three dimensions and/or is only over short distances. Another term for a non-crystalline form of a material is the "amorphous" form of the material. As previously discussed, the non-crystalline form of a low-solubility drug is preferred as it provides a greater aqueous concentration of drug relative to the crystalline form of the drug in an aqueous use environment. Preferably at least about 95 wt% of the drug in the nanoparticle is non-crystalline; in other words, the amount of drug in crystalline form does not exceed about 5 wt%. Amounts of crystalline drug may be measured by Powder X-Ray Diffraction (PXRD), by Differential Scanning Calorimetry (DSC), solid-state nuclear magnetic resonance (NMR), or by any other known quantitative measurement.

The nanoparticles can exist in a number of different configurations. In one embodiment, the nanoparticles comprise a core, the core comprising the non-crystalline drug and the poorly aqueous soluble non-ionizable polymer. As used herein, the term "core" refers to the interior portion of the nanoparticle. The nanoparticles also have a "surface portion," meaning the outside or exterior portion of the nanoparticle. Thus, the nanoparticles consist of a core (i.e., the interior portion) and a surface portion. In some embodiments, described herein below, materials may be adsorbed to the surface portion of the nanoparticle. Materials adsorbed to the surface portion of the nanoparticle are considered part of the nanoparticle, but are distinguishable from the core of the nanoparticle. Methods to distinguish materials present in the core versus materials adsorbed to the surface of the core include (1) thermal methods, such as differential scanning calorimetry (DSC); (2) spectroscopic methods, such as X-ray photoelectron spectroscopy (XPS), transmission electron microscopy (TEM) with energy dispersive X-ray (EDX) analysis, fourier transform infra red (FTIR) analysis, and raman spectroscopy; (3) chromatographic techniques, such as high performance liquid chromatography (HPLC), and gel-permeation chromatography (GPC); and (4) other techniques known in the art.

In one embodiment, the non-crystalline drug and the poorly aqueous soluble non-ionizable polymer constitute at least 60 wt% of the core, more preferably at least 80 wt% of the core. In another embodiment, the core consists essentially of the non-crystalline drug and the poorly aqueous soluble non-ionizable polymer.

The non-crystalline drug present in the core can exist in non-crystalline pure drug domains, as a thermodynamically stable solid solution of non-crystalline drug homogeneously distributed throughout the non-ionizable polymer, as a supersaturated solid solution of non-crystalline drug homogeneously distributed throughout the non-ionizable polymer, or any combination of these states or those states that lie between them. When the glass-transition temperature (T_{g}) of the non-crystalline drug is different from the T_{g} of the pure polymer by at least about 20°C, the core may exhibit a T_{g} that is between the T_{g} of pure non-crystalline drug or pure polymer. Preferably, less than 20 wt% of the drug is present in non-crystalline drug domains, with the remaining drug homogeneously distributed throughout the non-ionizable polymer.

In yet another embodiment, the core comprises the non-crystalline drug, the poorly aqueous soluble non-ionizable polymer, and an anionic cellulosic polymer. In this embodiment, the drug, non-ionizable polymer, and anionic cellulosic polymer constitute at least 60 wt% of the core, more preferably at least 80 wt% of the core.

The core may be (1) a homogeneous molecular mixture of drug, non-ionizable polymer, and anionic cellulosic polymer, (2) domains of pure drug, domains of pure non-ionizable polymer, and domains of pure anionic cellulosic polymer distributed throughout the core, or (3) any combination of these states or those states that lie between them. In one embodiment, the drug, non-ionizable polymer, and anionic cellulosic polymer are homogeneously distributed throughout the core as a supersaturated solid solution. In another embodiment, the surface portion of the nanoparticle has a higher concentration of anionic cellulosic polymer relative to the nanoparticle as a whole.

In still another embodiment, the core comprises the non-crystalline drug and the poorly aqueous soluble non-ionizable polymer, with the anionic cellulosic polymer adsorbed to the surface portion of the nanoparticle.

In yet another embodiment, the core comprises the non-crystalline drug, the poorly aqueous soluble non-ionizable polymer, and a portion of the anionic cellulosic polymer. The remaining portion of the anionic cellulosic polymer is adsorbed to the surface portion of the nanoparticle. In this embodiment, a portion of the anionic cellulosic polymer is integral to the core, while the remaining portion of anionic cellulosic polymer is adsorbed to the surface portion of the nanoparticle.

The drug and polymers are collectively present in the nanoparticle in an amount ranging from about 80 wt% to 100 wt%. Preferably, the drug and polymers collectively constitute at least 85 wt%, more preferably at least 90 wt% of the nanoparticle. In one embodiment, the nanoparticles consist essentially of the drug, the non-ionizable polymer and the anionic cellulosic polymer. By "consist essentially of" is meant that the nanoparticle contains less than 1 wt% of any other excipients and that any such excipients have no affect on the performance or properties of the nanoparticle.

The amount of drug in the nanoparticle may range from 0.1 wt% to 90 wt%. Preferably the amount of drug in the nanoparticle ranges from about 1 wt% to about 80 wt%, more preferably from about 5 wt% to about 75 wt%, even more preferably from about 10 wt% to about 60 wt%, and most preferably from about 10 wt% to about 50 wt%.

To minimize the total mass of the formulation, high drug loadings are desired. However, if the amount of drug in the nanoparticle is too high, the nanoparticle suspension becomes unstable, resulting in crystallization of the drug in the suspension. Additionally, high amounts of drug in the nanoparticle can lead to crystalline drug formation when the nanoparticles are isolated from suspension in solid form. In absolute terms, it is generally preferred that the amount of drug in the nanoparticle be less than about 90 wt%, more preferably less than about 80 wt%, even more preferably less than about 75 wt% the total mass of the nanoparticle.

The amount of poorly aqueous soluble non-ionizable polymer may range from 10 wt% to 75 wt%. The physical stability of the non-crystalline drug in the nanoparticle tends to improve with increasing amounts of the poorly aqueous soluble non-ionizable polymer. Accordingly, it is preferred that the amount of poorly aqueous soluble non-ionizable polymer in the nanoparticle is at least 15 wt%, more preferably at least 20 wt%, and most preferably at least 25 wt%. However, too much non-ionizable polymer will lead to low drug loading in the nanoparticle and low amounts of the anionic cellulosic polymer. Thus, it is preferred that the amount of poorly aqueous soluble non-ionizable polymer in the nanoparticle is 70% or less, and most preferably 60 wt% or less.

The amount of anionic cellulosic polymer may range from 5 wt% to 80 wt%. Preferably, the anionic cellulosic polymer is present in a sufficient amount so that the nanoparticles have sufficient surface charge so that they do not agglomerate into larger particles in solution. Preferably the amount of anionic cellulosic polymer in the nanoparticle is at least 10 wt%, more preferably at least 15 wt%, and most preferably at least 20 wt%. However, too much anionic cellulosic polymer will lead to low drug loading and low amounts of the non-ionizable polymer. Thus, it is preferred that the amount of anionic cellulosic polymer be 75 wt% or less, and most preferably 70 wt% or less.

In one embodiment, the mass ratio of non-ionizable polymer to anionic cellulosic polymer ranges from 1:8 to 15:1, more preferably from 1:6 to 9:1, even more preferably from 1:4 to 4:1, and most preferably from 1:3 to 3:1. Preferably, there is a sufficient amount of non-ionizable polymer to stabilize the drug in the nanoparticle, and a sufficient amount of anionic cellulosic polymer to stabilize the nanoparticles in solution.

Preferred embodiments of nanoparticles have the following amount of drug, poorly aqueous soluble non-ionizable polymer and anionic cellulosic polymer:
5 to 75 wt%, preferably 10 to 60 wt%, more preferably 20 to 50 wt% drug;
10 to 75 wt%, preferably 20 to 60 wt%, more preferably 25 to 60 wt% poorly aqueous soluble non-ionizable polymer; and
5 to 60 wt%, preferably 5 to 50 wt%, and more preferably 10 to 40 wt% anionic cellulosic polymer.

The nanoparticle is preferably substantially free from surfactants. By a "surfactant" is meant a surface-active material with a molecular weight of less than about 2000 daltons having a hydrophobic portion and a hydrophilic portion, and which is typically soluble in the use environment. By substantially "free from" is meant that the amount of surfactant present in the composition is less than 0.1 wt%. Preferably, the amount of the surfactant present in the nanoparticles is less than the detection limit.

Preferably, the nanoparticles are ionized when present in an aqueous use environment. It is believed that physical stability of the nanoparticles, in the sense of not aggregating or flocculating, is related, in part, to the amount of electric charge on the nanoparticle. To maximize the physical stability of the nanoparticle suspension, preferably the nanoparticles are at least partially ionized in the aqueous use environment. An indirect measure of charge is zeta potential. The nanoparticles preferably have a zeta potential of less than -10 mV (that is, the absolute value of the zeta potential is greater than 10 mV). Preferably, to reduce aggregation, the absolute value of the zeta potential is at least 20 mV, more preferably at least 30 mV, and even more preferably at least 40 mV. Zeta potential is typically calculated from the electrophoretic mobility measured by light scattering, R.J. Hunter, Zeta Potential in Colloid Science. Principles and Applications, Academic Press, 1981. Zeta potential may be measured using any number of commercially-available instruments, such as Brookhaven Instruments Corp. (Holtsville, NY) ZetaPals zeta potential analyzer.

### Non-ionizable Polymers

The nanoparticles of the present invention comprise a poorly aqueous soluble non-ionizable polymer. The term "polymer" is used conventionally, meaning a compound that is made of monomers connected together to form a larger molecule. A polymer generally consists of at least about 20 monomers connected together. Thus, the molecular weight of the polymer generally will be about 2000 daltons or more. The polymer should be inert, in the sense that it does not chemically react with the drug in an adverse manner, and should be pharmaceutically acceptable.

The polymer is a poorly aqueous soluble non-ionizable polymer. By "poorly aqueous soluble" is meant that the polymer has a solubility of less than 0.1 mg/mL when administered alone at a concentration of 0.2 mg/mL to a phosphate buffered saline solution (PBS) at pH 6.5. An appropriate PBS solution is an aqueous solution consisting of 20 mM sodium phosphate (Na₂HPO₄), 47 mM potassium phosphate (KH₂PO₄), 87 mM NaCl, and 0.2 mM KCI, adjusted to pH 6.5 with NaOH. A test to determine whether the polymer is poorly aqueous soluble may be performed as follows. The polymer is initially present in bulk powder form with average particle sizes of greater than about 1 micron. The polymer alone is administered at a concentration of 0.2 mg/mL to the PBS solution and stirred for approximately 1 hour at room temperature. Next, a nylon 0.45 µm filter is weighed, and the polymer solution is filtered. The filter is dried overnight at 40°C, and weighed the following morning. The amount of polymer dissolved is calculated from the amount of polymer added to the PBS solution minus the amount of polymer remaining on the filter (mg). The non-ionizable polymer is considered to be poorly aqueous soluble if it has a solubility of less than 0.1 mg/mL in this test. Preferably, when administered at a concentration of 0.2 mg/mL to the pH 6.5 PBS, a poorly aqueous soluble non-ionizable polymer has a solubility of less than 0.07 mg/mL, more preferably less than 0.05 mg/mL, and most preferably less than 0.01 mg/mL.

To ease processing, it is preferred that the poorly aqueous soluble non-ionizable polymer be soluble in an organic solvent. Preferably the polymer has a solubility in an organic solvent of at least about 0.1 mg/mL, and preferably at least 1 mg/mL. Preferably the polymer is not crosslinked.

The polymer is "non-ionizable," meaning that the polymer possesses substantially no ionizable functional groups. By "substantially no ionizable functional groups" is meant that the number of ionizable groups covalently attached to the polymer is less than about 0.05 milliequivalents per gram of polymer. Preferably, the number is less than about 0.02 milliequivalents per gram of polymer. By "ionizable groups" is meant functional groups that are at least about 10% ionized over at least a portion of the physiologically relevant pH range of 1 to 8. Such groups have pKₐ values of about 0 to 9.

Poorly aqueous soluble non-ionizable polymers for use with the present invention include substituted cellulosics, and non-cellulosics. By "cellulosic" is meant a cellulose polymer that has been modified by reaction of at least a portion of the hydroxyl groups on the saccharide repeating units with a compound to form an ester or an ether substituent.

In order to be poorly aqueous soluble, the polymer preferably has a sufficient number of hydrophobic groups relative to hydrophilic groups. In a preferred embodiment, the poorly aqueous soluble non-ionizable cellulosic has an ether- or ester-linked alkyl substituent. Suitable alkyl substituents include C₁ to C₄ alkyl groups. Exemplary ether-linked alkyl substituents include methyl, ethyl, propyl, and butyl groups. Exemplary ester-linked alkyl substituents include acetate, propionate, and butyrate groups.

Exemplary substituted cellulosics include methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, and hydroxypropyl methylcellulose butyrate. Preferably the poorly aqueous soluble non-ionizable polymer is selected from the group consisting of ethyl cellulose, cellulose acetate, cellulose propionate, cellulose butyrate, and cellulose acetate butyrate.

Exemplary non-cellulosics include vinyl polymers and copolymers, such as poly(vinyl acetate), poly(vinyl acetate-co-vinyl alcohol), and poly(ethylene-co-vinyl acetate); polymethacrylate and polyacrylate polymers and copolymers, such as poly(ethyl acrylate-co-methyl methacrylate), available as EUDRAGIT® NE; polylactones, such as poly(lactide), poly(glycolide), poly(ε-caprolactone), and copolymers of these, including poly(lactide-co-glycolide), poly(lactide-co-ε-caprolactone), poly(ethylene oxide-co-ε-caprolactone), poly(ethylene oxide-co-lactide), and poly(ethylene oxide-co-lactide-co-glycolide); and poly(alkyl)cyanoacrylates, such as poly(isobutyl)cyanoacrylate, and poly(hexyl)cyanoacrylate; and mixtures thereof.

In one embodiment, the non-ionizable polymer is selected from the group consisting of methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, hydroxypropyl methylcellulose butyrate, poly(vinyl acetate), poly(vinyl acetate-co-vinyl alcohol), poly(ethylene-co-vinyl acetate), poly(ethyl acrylate-co-methyl methacrylate) (available as EUDRAGIT® NE), poly(lactide), poly(glycolide), poly(ε-caprolactone), poly(lactide-co-glycolide), poly(lactide-co-ε-caprolactone), poly(ethylene oxide-co-ε-caprolactone), poly(ethylene oxide-co-lactide), poly(ethylene oxide-co-lactide-co-glycolide, poly(isobutyl)cyanoacrylate, and poly(hexyl)cyanoacrylate.

In another embodiment, the non-ionizable polymer is selected from the group consisting of methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, and hydroxypropyl methylcellulose butyrate.

In another embodiment, the non-ionizable polymer is selected from the group consisting of ethylcellulose, cellulose acetate, cellulose propionate, cellulose butyrate, and cellulose acetate butyrate.

In another embodiment, the non-ionizable polymer is ethylcellulose.

### Anionic Cellulosic Polymers

The nanoparticles also comprise an anionic cellulosic polymer. By "anionic cellulosic polymer" is meant that the cellulosic polymer has anionic substituents that are capable of being negatively charged at any pH over the physiologically relevant pH range of 1-8. Preferred anionic substituents include ether-linked alkyl carboxy groups, such as carboxy methyl and carboxy ethyl, and ester-linked substituents comprising a carboxylic acid group such as succinate, phthalate, trimellitate, and maleate. The number of anionic groups covalently attached to the polymer is preferably at least about 0.05 milliequivalents per gram of polymer. Preferably, the number is at least about 0.1 milliequivalents per gram of polymer.

It is preferred that the anionic cellulosic polymer be soluble in an organic solvent. Preferably the polymer has a solubility in an organic solvent of at least about 0.1 mg/mL, and preferably at least 1 mg/mL. Preferably the polymer is not crosslinked.

Suitable anionic cellulosic polymers include hydroxypropyl methyl cellulose acetate succinate (HPMCAS), hydroxypropyl methyl cellulose phthalate (HPMCP), carboxymethyl ethylcellulose (CMEC), carboxymethylcellulose acetate butyrate (CMCAB), cellulose acetate phthalate (CAP), hydroxypropyl methyl cellulose acetate phthalate (HPMCAP), cellulose acetate trimellitate (CAT), hydroxypropyl methyl cellulose acetate trimellitate (HPMCAT), ethyl cellulose succinate (ECS), ethyl cellulose phthalate (ECP), ethyl cellulose trimellitate (ECT), cellulose acetate succinate (CAS), and methylcellulose acetate succinate (MCAS).

A preferred subset of anionic cellulosic polymers includes hydroxypropyl methyl cellulose acetate succinate (HPMCAS), hydroxypropyl methyl cellulose phthalate (HPMCP), carboxymethyl ethylcellulose (CMEC), carboxymethylcellulose acetate butyrate (CMCAB), cellulose acetate phthalate (CAP), cellulose acetate trimellitate (CAT), ethyl cellulose succinate (ECS), and cellulose acetate succinate (CAS).

In one embodiment, the anionic cellulosic polymer is HPMCAS.

In another embodiment, the anionic cellulosic polymer is CMEC.

In another embodiment, the anionic cellulosic polymer is CMCAB.

In another embodiment, the anionic cellulosic polymer is CAP.

In another embodiment, the anionic cellulosic polymer is ECS.

In yet another embodiment, the non-ionizable polymer is selected from the group consisting of methylcellulose ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate, low-substituted hydroxypropyl methylcellulose, hydroxypropyl methylcellulose propionate, hydroxypropyl methylcellulose butyrate, poly(vinyl acetate), poly(vinyl acetate-co-vinyl alcohol), poly(ethylene-co-vinyl acetate), poly(ethyl acrylate-co-methyl methacrylate), poly(lactide), poly(glycolide), poly(ε-caprolactone), poly(lactide-co-glycolide), poly(lactide-co-ε-caprolactone), poly(ethylene oxide-co-ε-caprolactone), poly(ethylene oxide-co-lactide), poly(ethylene oxide-co-lactide-co-glycolide, poly(isobutyl)cyanoacrylate, and poly(hexyl)cyanoacrylate, and the anionic cellulosic polymer is selected from the group consisting of hydroxypropyl methyl cellulose acetate succinate (HPMCAS), hydroxypropyl methyl cellulose phthalate (HPMCP), carboxymethyl ethylcellulose (CMEC), carboxymethylcellulose acetate butyrate (CMCAB), cellulose acetate phthalate (CAP), hydroxypropyl methyl cellulose acetate phthalate (HPMCAP), cellulose acetate trimellitate (CAT), hydroxypropyl methyl cellulose acetate trimellitate (HPMCAT), ethyl cellulose succinate (ECS), ethyl cellulose phthalate (ECP), ethyl cellulose trimellitate (ECT), cellulose acetate succinate (CAS), and methylcellulose acetate succinate (MCAS).

In yet another embodiment, the non-ionizable polymer is selected from the group consisting of methylcellulose ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, hydroxypropyl methylcellulose acetate, low-substituted hydroxypropyl methylcellulose, hydroxypropyl methylcellulose propionate, hydroxypropyl methylcellulose butyrate, and the anionic cellulosic polymer is selected from the group consisting of hydroxypropyl methyl cellulose acetate succinate (HPMCAS), hydroxypropyl methyl cellulose phthalate (HPMCP), carboxymethyl ethylcellulose (CMEC), carboxymethylcellulose acetate butyrate (CMCAB), cellulose acetate phthalate (CAP), hydroxypropyl methyl cellulose acetate phthalate (HPMCAP), cellulose acetate trimellitate (CAT), hydroxypropyl methyl cellulose acetate trimellitate (HPMCAT), ethyl cellulose succinate (ECS), ethyl cellulose phthalate (ECP), ethyl cellulose trimellitate (ECT), cellulose acetate succinate (CAS), and methylcellulose acetate succinate (MCAS).

In still another embodiment, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of hydroxypropyl methyl cellulose acetate succinate (HPMCAS), hydroxypropyl methyl cellulose phthalate (HPMCP), carboxymethyl ethylcellulose (CMEC), carboxymethylcellulose acetate butyrate (CMCAB), cellulose acetate phthalate (CAP), hydroxypropyl methyl cellulose acetate phthalate (HPMCAP), cellulose acetate trimellitate (CAT), hydroxypropyl methyl cellulose acetate trimellitate (HPMCAT), ethyl cellulose succinate (ECS), ethyl cellulose phthalate (ECP), ethyl cellulose trimellitate (ECT), cellulose acetate succinate (CAS), and methylcellulose acetate succinate (MCAS).

In yet another embodiment, the non-ionizable polymer is ethylcellulose, and the anionic cellulosic polymer is selected from the group consisting of HPMCAS, HPMCP, CMEC, CMCAB, CAP, HPMCAP, CAT, HPMCAT, ECS, ECP, ECT, CAS, and MCAS.

In another embodiment, the non-ionizable polymer is selected from the group consisting of methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, hydroxypropyl methylcellulose butyrate, poly(vinyl acetate), poly(vinyl acetate-co-vinyl alcohol), poly(ethylene-co-vinyl acetate), poly(ethyl acrylate-co-methyl methacrylate), poly(lactide), poly(glycolide), poly(ε-caprolactone), poly(lactide-co-glycolide), poly(lactide-co-ε-caprolactone), poly(ethylene oxide-co-ε-caprolactone), poly(ethylene oxide-co-lactide), poly(ethylene oxide-co-lactide-co-glycolide, poly(isobutyl)cyanoacrylate, and poly(hexyl)cyanoacrylate, and the anionic cellulosic polymer is selected from the group consisting of HPMCAS, HPMCP, CMEC, CMCAB, CAP, CAT, ECS, and CAS.

In another embodiment, the non-ionizable polymer is selected from the group consisting of methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, hydroxypropyl methylcellulose butyrate, and the anionic cellulosic polymer is selected from the group consisting of HPMCAS, HPMCP, CMEC, CMCAB, CAP, CAT, ECS, and CAS.

In another embodiment, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of HPMCAS, HPMCP, CMEC, CMCAB, CAP, CAT, ECS, and CAS.

In still another embodiment, non-ionizable polymer is selected from the group consisting of ethylcellulose, cellulose acetate, and cellulose acetate butyrate, and the anionic cellulosic polymer is selected from the group consisting of hydroxypropyl methyl cellulose acetate succinate (HPMCAS), carboxymethyl ethylcellulose (CMEC), carboxymethylcellulose acetate butyrate (CMCAB), cellulose acetate phthalate (CAP), and ethyl cellulose succinate (ECS).

In still another embodiment, the non-ionizable polymer is ethylcellulose, and the anionic cellulosic polymer is selected from the group consisting of HPMCAS, HPMCP, CMEC, CMCAB, CAP, CAT, ECS, and CAS.

In still another embodiment, the non-ionizable polymer is ethylcellulose, and the anionic cellulosic polymer is selected from the group consisting of HPMCAS, ECS, and CMCAB.

### The Drug

The drug is a "poorly water soluble drug," meaning that the drug has a solubility in water (over the pH range of 6.5 to 7.5 at 25 °C) of less than 5 mg/mL. The utility of the invention increases as the water solubility of the drug decreases. The drug may have an even lower solubility in water, such as less than about 1 mg/mL, less than about 0.1 mg/mL, and even less than about 0.01 mg/mL.

In general, it may be said that the drug has a dose-to-aqueous solubility ratio greater than about 10 mL, and more typically greater than about 100 mL, where the aqueous solubility (mg/mL) is the minimum value observed in any physiologically relevant aqueous solution (i.e., solutions with pH 1- 8), including USP simulated gastric and intestinal buffers, and dose is in mg. Thus, a dose-to-aqueous solubility ratio may be calculated by dividing the dose (in mg) by the aqueous solubility (in mg/mL).

Preferred classes of drugs include, but are not limited to, compounds for use in the following therapeutic areas: antihypertensives, antianxiety agents, antiarrythmia agents, anticlotting agents, anticonvulsants, blood glucose-lowering agents, decongestants, antihistamines, antitussives, antineoplastics, beta blockers, anti-inflammatories, antipsychotic agents, cognitive enhancers, anti-atherosclerotic agents, cholesterol-reducing agents, triglyceride-reducing agents, antiobesity agents, autoimmune disorder agents, anti-impotence agents, antibacterial and antifungal agents, hypnotic agents, anti-Parkinsonism agents, anti-Alzheimer's disease agents, antibiotics, anti-angiogenesis agents, anti-glaucoma agents, anti-depressants, and antiviral agents.

Each named drug should be understood to include the neutral form of the drug or pharmaceutically acceptable forms of the drug. By "pharmaceutically acceptable forms" is meant any pharmaceutically acceptable derivative or variation, including stereoisomers, stereoisomer mixtures, enantiomers, solvates, hydrates, isomorphs, polymorphs, pseudomorphs, neutral forms, salt forms and prodrugs.

Exemplary drugs suitable for use in the nanoparticles include, but are not limited to, phosphodiesterase inhibitors, such as sildenafil and sildenafil citrate; HMG-CoA reductase inhibitors, such as atorvastatin, lovastatin, simvastatin, pravastatin, fluvastatin, rosuvastatin, itavastatin, nisvastatin, visastatin, atavastatin, bervastatin, compactin, dihydrocompactin, dalvastatin, fluindostatin, pitivastatin, and velostatin (also referred to as synvinolin); vasodilator agents, such amiodarone; antipsychotics, such as ziprasidone; calcium channel blockers, such as nifedipine, nicardipine, verapamil, and amlodipine; cholesteryl ester transfer protein (CETP) inhibitors; cyclooxygenase-2 inhibitors; microsomal triglyceride transfer protein (MTP) inhibitors; vascular endothelial growth factor (VEGF) receptor inhibitors; carbonic anhydrase inhibitors; and glycogen phosphorylase inhibitors. Other low-solubility drugs suitable for use in the nanoparticles are disclosed in US Published patent application 2005/0031692, herein incorporated by reference.

In one embodiment, the drug is ziprasidone or a pharmaceutically acceptable form thereof.

In one embodiment, the drug is a hydrophobic non-ionizable drug. By "hydrophobic non-ionizable drug" is meant a subclass of non-ionizable drugs that are essentially water insoluble and highly hydrophobic, and are characterized by a set of physical properties, as described hereinafter. By "non-ionizable" is meant that the drug has substantially no ionizable groups. By "ionizable groups" is meant functional groups that are at least about 10% ionized over at least a portion of the physiologically relevant pH range of 1 to 8. Such groups have pKa values of about 0 to 9. Thus, hydrophobic non-ionizable drugs do not have a pKa value between 0 and 9.

The first property of hydrophobic drugs is that they are extremely hydrophobic. Log P, defined as the base 10 logarithm of the ratio of the drug solubility in octanol to the drug solubility in water, is a widely accepted measure of hydrophobicity. By "extremely hydrophobic" is meant that the Log P value of the drug is at least 4.0, preferably at least 4.5, and most preferably at least 5.0. Log P may be measured experimentally or calculated using methods known in the art. When using a calculated value for Log P, the highest value calculated using any generally accepted method for calculating Log P is used. Calculated Log P values are often referred to by the calculation method, such as Clog P, Alog P, and Mlog P. The Log P may also be estimated using fragmentation methods, such as Crippen's fragmentation method (27 J.Chem.Inf.Comput.Sci. 21 (1987)); Viswanadhan's fragmentation method (29 J.Chem.Inf.Comput.Sci. 163 (1989)); or Broto's fragmentation method (19 Eur.J.Med.Chem.-Chim.Theor. 71 (1984). Preferably the Log P value is calculated by using the average value estimated using Crippen's, Viswanadhan's, and Broto's fragmentation methods.

The second property of hydrophobic drugs is that they have an extremely low solubility in water over the pH range of 6.5 to 7.5 at 25°C. By "extremely low solubility in water" is meant that the solubility of the drug in water is less than 100 µg/mL. Preferably, the hydrophobic drug has a water solubility of less than 50 µg/mL, and most preferably less than 10 µg/mL.

In one embodiment, the nanoparticles comprise from 20 wt% to 40 wt% of a hydrophobic drug, from 30 wt% to 45 wt% of a non-ionizable polymer, and from 15 wt% to 50 wt% of an anionic cellulosic polymer. Preferably, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, cellulose acetate succinate, polyvinyl acetate phthalate, vinyl acetate-maleic acid copolymer, poly[methacrylic acid-co-methyl methacrylate], poly[methacrylic acid-co-ethyl acrylate], and poly(methyl methacrylate-co-2-acrylamido-2-methylpropane sulfonic acid).

In another embodiment the drug is a cholesteryl ester transfer protein (CETP) inhibitor. CETP inhibitors are drugs that inhibit CETP activity. The effect of a drug on the activity of CETP can be determined by measuring the relative transfer ratio of radiolabeled lipids between lipoprotein fractions, essentially as previously described by Morton in J. Biol. Chem. 256, 11992, 1981 and by Dias in Clin. Chem. 34, 2322, 1988, and as presented in U.S. Patent No. 6,197,786, the disclosures of which are herein incorporated by reference. The potency of CETP inhibitors may be determined by performing the above-described assay in the presence of varying concentrations of the test compounds and determining the concentration required for 50% inhibition of transfer of radiolabeled lipids between lipoprotein fractions. This value is defined as the "IC₅₀ value." Preferably, the CETP inhibitor has an IC₅₀ value of less than about 2000 nM, more preferably less than about 1500 nM, even more preferably less than about 1000 nM, and most preferably less than about 500 nM.

Specific examples of CETP inhibitors include [2R,4S] 4-[acetyl-(3,5-bis-trifluoromethyl-benzyl)-amino]-2-ethyl-6-trifluoromethyl-3,4-dihydro-2H-quinoline-1-carboxylic acid isopropyl ester; (2R)-3-[[3-(4-chloro-3-ethylphenoxy)phenyl][[3-(1,1,2,2-tetrafluoroethoxy)phenyl]methyl]amino]-1,1,1-trifluoro-2-propanol; S-[2-([[1-(2-ethylbutyl)cyclohexyl]carbonyl]amino)phenyl]2-methylpropanethioate; trans-4-[[[2-[[[[3,5-bis(trifluoromethyl)phenyl]methyl](2-methyl-2H-tetrazol-5-yl)amino]methyl]-4-(trifluoromethyl)phenyl]ethylamino]methyl]-cyclohexaneacetic acid; trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate; trans-(2R,4S)- 2-(4-{4-[(3,5-bis-trifluoromethyl-benzyl)-(2-methyl-2H-tetrazol-5-yl)-amino]-2-ethyl-6-trifluoromethyl-3,4-dihydro-2H-quinoline-1-carbonyl}-cyclohexyl)-acetamide; methyl N-(3-cyano-5-trifluoromethylbenzyl)-[6-(N'-cyclopentylmethyl-N'-ethylamino)indan-5-ylmethyl]-carbamate; methyl (3-cyano-5-trifluoromethylbenzyl)-[6-(N-cyclopentylmethyl-N-ethylamino)indan-5-ylmethyl]-carbamate; ethyl 4-((3,5-bis(trifluoromethyl)phenyl)(2-methyl-2H-tetrazol-5-yl)methyl)-2-ethyl-6-(trifluoromethyl)-3,4-dihydroquinoxaline-1 (2H)-carboxylate; tert-butyl 5-(N-(3,5-bis(trifluoromethyl)benzyl)acetamido)-7-methyl-8-(trifluoromethyl)-2,3,4,5-tetrahydrobenzo[b]azepine-1-carboxylate; (3,5-bis-trifluoromethyl-benzyl)-[2-(cyclohexyl-methoxy-methyl)-5-trifluoromethyl-benzyl]-(2-methyl-2H-tetrazol-5-yl)-amine; 1-[1-(2-{[(3,5-bis-trifluoromethyl-benzyl)-(2-methyl-2H-tetrazol-5-yl)-amino]-methyl}-4-trifluoromethyl-phenyl)-2-methyl-propyl]-piperidine-4-carboxylic acid; (3,5-bis-trifluoromethyl-benzyl)-[2-(1-methoxy-cycloheptyl)-5-trifluoromethyl-benzyl]-(2-methyl-2H-tetrazol-5-yl)-amine; (3,5-bis-trifluoromethyl-benzyl)-[2-(1-cyclohexyl-1-methoxy-ethyl)-5-trifluoromethyl-benzyl]-(2-methyl-2H-tetrazol-5-yl)-amine; the drugs disclosed in commonly owned U.S. Patent Application Serial Nos. 09/918,127 and 10/066,091, the disclosures of both of which are incorporated herein by reference; and the drugs disclosed in the following patents and published applications, the disclosures of all of which are incorporated herein by reference: DE 19741400 A1; DE 19741399 A1; WO 9914215 A1; WO 9914174; DE 19709125 A1; DE 19704244 A1; DE 19704243 A1; EP 818448 A1; WO 9804528 A2; DE 19627431 A1; DE 19627430 A1; DE 19627419 A1; EP 796846 A1; DE 19832159; DE 818197; DE 19741051; WO 9941237 A1; WO 9914204 A1; JP 11049743; WO 0018721; WO 0018723; WO 0018724; WO 0017164; WO 0017165; WO 0017166; EP 992496; EP 987251; WO 9835937; JP 03221376; WO 04020393; WO 05095395; WO 05095409; WO 05100298; WO 05037796; WO 0509805; WO 03028727; WO 04039364; WO 04039453; WO 0633002; and U.S. Provisional Patent Application Numbers 60/781488 and 60/780993, both of which were filed on March 10, 2006.

Thus, in one embodiment, the CETP inhibitor is selected from the group of compounds mentioned above. In another embodiment, the CETP inhibitor is selected from the group consisting of (2R)-3-[[3-(4-chloro-3-ethylphenoxy)phenyl][[3-(1,1,2,2-tetrafluoroethoxy)phenyl]methyl]amino]-1,1,1-trifluoro-2-propanol; trans-(2R,4S)- 2-(4-{4-[(3,5-bis-trifluoromethyl-benzyl)-(2-methyl-2H-tetrazol-5-yl)-amino]-2-ethyl-6-trifluoromethyl-3,4-dihydro-2H-quinoline-1-carbonyl}-cyclohexyl)-acetamide; (3,5-bis-trifluoromethyl-benzyl)-[2-(cyclohexyl-methoxy-methyl)-5-trifluoromethylbenzyl]-(2-methyl-2H-tetrazol-5-yl)-amine; 1-[1-(2-{[(3,5-bis-trifluoromethyl-benzyl)-(2-methyl-2H-tetrazol-5-yl)-amino]-methyl}-4-trifluoromethyl-phenyl)-2-methyl-propyl]-piperidine-4-carboxylic acid; (3,5-bis-trifluoromethyl-benzyl)-[2-(1-methoxy-cycloheptyl)-5-trifluoromethyl-benzyl]-(2-methyl-2H-tetrazol-5-yl)-amine; (3,5-bis-trifluoromethyl-benzyl)-[2-(1-cyclohexyl-1-methoxy-ethyl)-5-trifluoromethyl-benzyl]-(2-methyl-2H-tetrazol-5-yl)-amine, and pharmaceutically acceptable forms thereof.

In still another embodiment, the CETP inhibitor is (2R)-3-[[3-(4-chloro-3-ethylphenoxy)phenyl][[3-(1,1,2,2-tetrafluoroethoxy) phenyl]methyl]amino]-1,1,1-trifluoro-2-propanol.

In still another embodiment, the CETP inhibitor is trans-(2R,4S)- 2-(4-{4-[(3 15-Bis-trifluoromethyl-benzyl)-(2-methyl-2H-tetrazol-5-yl)-amino]-2-ethyl-6-trifluoromethyl-3,4-dihydro-2H-quinoline-1-carbonyl}-cyclohexyl)-acetamide.

In yet another embodiment, the nanoparticles comprise from 20 wt% to 40 wt% of a CETP inhibitor, from 30 wt% to 45 wt% of a non-ionizable polymer, and from 15 wt% to 50 wt% of an anionic cellulosic polymer. Preferably, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, cellulose acetate succinate, polyvinyl acetate phthalate, vinyl acetate-maleic acid copolymer, poly[methacrylic acid-co-methyl methacrylate], poly[methacrylic acid-co-ethyl acrylate], and poly(methyl methacrylate-co-2-acrylamido-2-methylpropane sulfonic acid).

In another embodiment, the drug is an inhibitor of cyclooxygenase-2 (COX-2). COX-2 inhibitors are nonsteroidal anti-inflammatory drugs that exhibit anti-inflammatory, analgesic and antipyretic effects. Preferably, the COX-2 inhibitor is a selective COX-2 inhibitor, meaning that the drug is able to inhibit COX-2 without significant inhibition of cyclooxygenase-1 (COX-1). Preferably, the COX-2 inhibitor has a potency such that the concentration of drug that inhibits 50% of COX-2 enzyme in an *in vitro* test (i.e., the IC₅₀ value) is less than about 10 µM, preferably less than 5 µM, more preferably less than 2 µM. In addition, it is also preferable that the COX-2 inhibitor be selective relative to COX-1. Thus, preferably, the ratio of the IC₅₀,_{COX-2} to IC_{50,COX-1} ratio for the compound is less than 0.5, more preferably less than 0.3, and most preferably less than 0.2.

Specific examples of COX-2 inhibitors include 4-(5-(4-methylphenyl)-3-(trifluoromethyl)-1H-pyrazol-1-yl)benzenesulfonamide (celecoxib); 4-(5-methyl)-3-phenylisoxazol-4-yl)benzenesulfonamide (valdecoxib); N-(4-(5-methyl-3-phenylisoxazol-4-yl)phenylsulfonyl)propionamide (paracoxb); sodium (S)-6,8-dichloro-2-(trifluoromethyl)-2H-chromene-3-carboxylate; sodium (S)-7-tert-butyl-6-chloro-2-(trifluoromethyl)-2H-chromene-3-carboxylate; 2-[(2-chloro-6-fluorophenyl)amino]-5-methyl benzeneacetic acid (lumiracoxib); 4-(3-(difluoromethyl)-5-(3-fluoro-4-methoxyphenyl)-1H-pyrazol-1-yl)benzenesulfonamide (deracoxib); 4-(4-(methylsulfonyl)phenyl)-3-phenylfuran-2(5H)-one (rofecoxib); 5-chloro-2-(6-methylpyridin-3-yl)-3-(4-(methylsulfonyl) phenyl)pyridine (etoricoxib); 2-(3,4-difluorophenyl)-4-(3-hydroxy-3-methylbutoxy)-5-(4-(methylsulfonyl)phenyl)pyridazin-3(2H)-one; (Z)-3-((3-chlorophenyl)(4-(methylsulfonyl)phenyl)methylene)-dihydrofuran-2(3H)-one; N-(2-(cyclohexyloxy)-4-nitrophenyl)methanesulfonamide; 4-Methyl-2-(3,4-dimethylphenyl)-1-(4-sulfamoyl-phenyl)-1H-pyrrole; 6-((5-(4-chlorobenzoyl)-1,4-dimethyl-1H-pyrrol-2-yl)methyl)pyridazin-3(2H)-one; 4-(4-cyclohexyl-2-methyloxazol-5-yl)-2-fluorobenzenesulfonamide (tilmacoxib); 2-(4-Ethoxyphenyl)-4-methyl-1-(4-sulfamoylphenyl)-1H-pyrrole; 4-hydroxy-2-methyl-N-(5-methyl-2-thiazolyl)-2H-1,2-benzothiazine-3-carboxamide-1,1-dioxide (meloxicam); 4-(4-chloro-5-(3-fluoro-4-methoxyphenyl)-1H-pyrazol-1-yl)benzenesulfonamide(cimicoxib), and pharmaceutically acceptable forms thereof; and the compounds disclosed in the following patents and published applications, the disclosures of which are incorporated herein by reference: US 5,466,823, US 5,633,272, US 5,932,598, US 6,034,256, US 6,180,651, US 5,908,858, US 5,521,207, US 5,691,374, WO 99/11605, WO 98/03484, and WO 00/24719.

Preferably the COX-2 inhibitor is selected from the group consisting of celecoxib; valdecoxib; paracoxb; sodium (S)-6,8-dichloro-2-(trifluoromethyl)-2H-chromene-3-carboxylate; sodium (S)-7-tert-butyl-6-chloro-2-(trifluoromethyl)-2H-chromene-3-carboxylate; and pharmaceutically acceptable forms thereof. In one embodiment, the COX-2 inhibitor is celecoxib or pharmaceutically acceptable forms thereof.

In another embodiment, the nanoparticles comprise from 20 wt% to 50 wt% of a COX-2 inhibitor, from 20 wt% to 60 wt% of a non-ionizable polymer, and from 5 wt% to 60 wt% of an anionic cellulosic polymer. Preferably, the non-ionizable polymer is selected from the group consisting of ethylcellulose, cellulose acetate, cellulose propionate, cellulose butyrate, and cellulose acetate butyrate, and the anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, cellulose acetate succinate, polyvinyl acetate phthalate, vinyl acetate-maleic acid copolymer, poly[methacrylic acid-co-methyl methacrylate], poly[methacrylic acid-co-ethyl acrylate], and poly(methyl methacrylate-co-2-acrylamido-2-methylpropane sulfonic acid).

### Process for Making Nanoparticles

The nanoparticles may be formed by any process that results in formation of nanoparticles of non-crystalline drug, a non-ionizable polymer, and an anionic cellulosic polymer. The drug used to form the nanoparticles may be in a crystalline or non-crystalline form; however, at least 90 wt% of the drug in the resulting nanoparticles is in non-crystalline form.

One process for forming nanoparticles is an emulsification process. In this process, the drug and polymers are dissolved in an organic solvent that is immiscible with an aqueous solution in which the drug and polymers are poorly soluble, forming an organic solution. Solvents suitable for forming the organic solution of dissolved drug and polymers can be any compound or mixture of compounds in which the drug and the polymer are mutually soluble and which is immiscible with the aqueous solution. As used herein, the term "immiscible" means that the organic solvent has a solubility in the aqueous solution of less than about 10 wt%, preferably less than about 5 wt%, and most preferably less than about 3 wt%. Preferably, the organic solvent is also volatile with a boiling point of 150°C or less. Exemplary solvents include methylene chloride, trichloroethylene, tetrachloroethane, trichloroethane, dichloroethane, dibromoethane, ethyl acetate, phenol, chloroform, toluene, xylene, ethyl-benzene, methyl-ethyl ketone, methyl-isobutyl ketone, and mixtures thereof. Preferred solvents are methylene chloride, ethyl acetate, benzyl alcohol, and mixtures thereof. The aqueous solution is preferably water.

Once the organic solution is formed, it is then mixed with the aqueous solution and homogenized to form an emulsion of fine droplets of the water immiscible solvent distributed throughout the aqueous phase. The volume ratio of organic solution to aqueous solution used in the process will generally range from 1:100 (organic solution:aqueous solution) to 2:3 (organic solution:aqueous solution). Preferably, the organic solution:aqueous solution volume ratio ranges from 1:9 to 1:2 (organic solution:aqueous solution). The emulsion is generally formed by a two-step homogenization procedure. The solution of drug, polymer and organic solvent are first mixed with the aqueous solution using a rotor/stator or similar mixer to create a "pre-emulsion". This mixture is then further processed with a high-pressure homogenizer that subjects the droplets to very high shear, creating a uniform emulsion of very small droplets. A portion of the solvent is then removed forming a suspension of the nanoparticles in the aqueous solution. Exemplary processes for removing the organic solvent include evaporation, extraction, diafiltration, pervaporation, vapor permeation, distillation, and filtration. Preferably, the solvent is removed to a level that is acceptable according to The International Committee on Harmonization (ICH) guidelines. Preferably, the concentration of solvent in the nanoparticle suspension is less than the solubility of the solvent in the aqueous solution. Even lower concentrations of solvent are preferred. Thus, the concentration of solvent in the nanoparticle suspension may be less than about 5 wt%, less than about 3 wt%, less than 1 wt%, and even less than 0.1 wt%.

Alternatively, the drug and non-ionizable polymer are dissolved in an organic solvent that is immiscible with the aqueous solution, while the anionic cellulosic polymer is dissolved or suspended in the aqueous solution. The nanoparticles are then formed using the above emulsification procedure.

An alternative process to form the nanoparticles is a precipitation process. In this process, the drug and polymers are first dissolved in an organic solvent that is miscible with an aqueous solution in which the drug and polymers are poorly soluble to form an organic solution. The organic solution is mixed with the aqueous solution causing the nanoparticles to precipitate. Solvents suitable for forming the organic solution of dissolved drug and polymers can be any compound or mixture of compounds in which the drug and the polymer are mutually soluble and which is miscible with the aqueous solution. Preferably, the organic solvent is also volatile with a boiling point of 150°C or less. In addition, the organic solvent should have relatively low toxicity. Exemplary solvents include acetone, methanol, ethanol, tetrahydrofuran (THF), and dimethylsulfoxide (DMSO). Mixtures of solvents, such as 50% methanol and 50% acetone, can also be used, so long as the polymer and drug are sufficiently soluble to dissolve the drug and polymer. Preferred solvents are methanol, acetone, and mixtures thereof.

The aqueous solution may be any compound or mixture of compounds in which the drug and polymers are sufficiently insoluble so as to precipitate to form nanoparticles. The aqueous solution is preferably water.

The organic solution and aqueous solution are combined under conditions that cause solids to precipitate as nanoparticles. The mixing can be by addition of a bolus or stream of organic solution to a stirring container of the aqueous solution. Alternately a stream or jet of organic solution can be mixed with a moving stream of aqueous solution. In either case, the precipitation results in the formation of a suspension of nanoparticles in the aqueous solution.

Alternatively, the drug and non-ionizable polymer are dissolved in the organic solvent, while the anionic cellulosic polymer is added to the aqueous solution. The nanoparticles are then formed using the above precipitation procedure.

For the precipitation process, the amount of drug and polymers in the organic solution depends on the solubility of each in the organic solvent and the desired ratios of drug to polymers in the resulting nanoparticles. The organic solution may comprise from about 0.1 wt% to about 20 wt% dissolved solids. A dissolved solids content of from about 0.5 wt% to 10 wt% is preferred.

The organic solution:aqueous solution volume ratio should be selected such that there is sufficient aqueous solution in the nanoparticle suspension that the nanoparticles solidify and do not rapidly agglomerate. However, too much aqueous solution will result in a very dilute suspension of nanoparticles, which may require further processing for ultimate use. Generally, the organic solution:aqueous solution volume ratio should be at least 1:100, but generally should be less than 1:2 (organic solution:aqueous solution). Preferably, the organic solution:aqueous solution volume ratio ranges from about 1:20 to about 1:3.

Once the nanoparticle suspension is made, a portion of the organic solvent may be removed from the suspension using methods known in the art. Exemplary processes for removing the organic solvent include evaporation, extraction, diafiltration, pervaporation, vapor permeation, distillation, and filtration. Preferably, the solvent is removed to a level that is acceptable according to ICH guidelines. Thus, the concentration of solvent in the nanoparticle suspension may be less than about 10 wt%, less than about 5 wt%, less than about 3 wt%, less than 1 wt%, and even less than 0.1 wt%.

Thus, in one embodiment, a process for forming nanoparticles, comprises: (a) forming an organic solution comprising a poorly water soluble drug and a poorly aqueous soluble non-ionizable polymer dissolved in an organic solvent, wherein the drug has a solubility in water of less than 5 mg/mL over the pH range of pH 6.5 to 7.5; (b) forming an aqueous solution, wherein the drug and the non-ionizable polymer are poorly soluble in the aqueous solution; (c) adding an anionic cellulosic polymer to at least one of the organic solution and the aqueous solution; (d) mixing the organic solution with the aqueous solution to form a first mixture; (e) removing the solvent from the first mixture to form a suspension comprising the nanoparticles and the aqueous solution, wherein (i) the nanoparticles have an average size of less than 500 nm, (ii) at least 90 wt% of the drug in the nanoparticles is non-crystalline, and (iii) the drug, the non-ionizable polymer, and the anionic cellulosic polymer collectively constitute at least 80 wt% of the nanoparticles.

Both the emulsion process and the precipitation process result in the formation of a suspension of the nanoparticles in the aqueous solution. In some instances it is desirable to concentrate the nanoparticles or to isolate the nanoparticles in solid form by removing some or all of the liquid from the suspension. Exemplary processes for removing at least a portion of the liquid include spray drying, spray coating, spray layering, lyophylization, evaporation, vacuum evaporation, filtration, ultrafiltration, reverse osmosis, and other processes known in the art. A preferred process is spray drying. One or more processes may be combined to remove the liquid from the nanoparticle suspension to yield a solid composition. For example, a portion of the liquids may be removed by filtration to concentrate the nanoparticles, followed by spray-drying to remove most of the remaining liquids, followed by a further drying step such as tray-drying.

When isolating the nanoparticles in solid form, it is often desirable to include a matrix material in the suspension of nanoparticles prior to removal of the liquids. The matrix material functions to help slow or prevent agglomeration of the nanoparticles as the liquids are being removed, as well as to help re-suspend the nanoparticles when the solid composition is added to an aqueous solution (e.g., an aqueous environment of use). The matrix material is preferably pharmaceutically acceptable and water soluble. Examples of matrix materials include polyvinyl pyrrolidone (PVP), trehalose, hydroxypropyl methyl cellulose (HPMC), hydroxypropyl cellulose (HPC), casein, caseinate, albumin, gelatin, acacia, lactose, mannitol, pharmaceutically acceptable forms thereof, and other matrix materials know in the art.

In one embodiment of the invention, a solid composition comprises (a) a plurality of nanoparticles comprising a poorly water-soluble drug, a non-ionizable polymer, and an anionic cellulosic polymer, and (b) a matrix material. As used herein, the term "solid pharmaceutical composition" means that the composition is in a solid form and substantially free of liquids. The nanoparticles are entrapped or encapsulated in the matrix material.

The presence of nanoparticles in the solid composition can be determined using the following procedure. A sample of the solid composition is embedded in a suitable material, such as an epoxy or polyacrylic acid (e.g., LR White from London Resin Co., London, England). The sample is then microtomed to obtain a cross-section of the solid composition that is about 100 to 200 nm thick. This sample is then analyzed using transmission electron microscopy (TEM) with energy dispersive X-ray (EDX) analysis. TEM-EDX analysis quantitatively measures the concentration and type of atoms larger than boron over the surface of the sample. From this analysis, regions that are rich in drug can be distinguished from regions that are rich in the matrix material. The size of the regions that are rich in drug will have an average diameter of less than 500 nm in this analysis, demonstrating that the solid composition comprises nanoparticles of drug in the matrix material. See, for example, *Transmission Electron Microscopy and Diffractometry of Materials* (2001) for further details of the TEM-EDX method.

Another procedure that demonstrates the solid composition contains nanoparticles is to administer a sample of the solid composition to water to form a suspension of the nanoparticles. The suspension is then analyzed by DLS as described herein. A solid composition of the invention will form nanoparticles having an average cumulant diameter of less than 500 nm.

A specific procedure for demonstrating the solid composition contains nanoparticles is as follows. A sample of the solid composition is added to water at ambient temperature such that the concentration of solids is less than about 1 mg/mL. The so-formed suspension is then analyzed by DLS. The solid composition contains nanoparticles if the DLS analysis results in particles having an average cumulant diameter of less than 500 nm.

A solid composition of the invention will show the presence of nanoparticles in at least one, and preferably both of the above tests.

### Resuspendability

In one embodiment, solid compositions of the present invention result in improved resuspendability of the nanoparticles relative to surfactant-based stabilizers. The term "resuspendability" as used herein means the ability of the solid material, when administered to an aqueous use environment, to form a nanoparticle suspension.

The ability of the solid composition to resuspend nanoparticles when administered to an aqueous solution can be determined using the following procedures. In the first procedure, the average particle size of the re-suspended material is determined as follows. The solid composition is added to an aqueous solution, such as water, PBS, or a model fasted duodenal (MFD) solution, to form a suspension. A sample of the solid composition is added to water at ambient temperature such that the concentration of solids is less than about 1 mg/mL. The average particle size of the nanoparticles formed during this (re)suspension is then determined by DLS techniques. A solid composition is said to provide good resuspendability if, upon administration to an aqueous solution, the average particle size as determined by DLS techniques is at least 50% and no more than 200% the average particle size of the nanoparticles prior to recovery of the solid composition. Preferably, the formulation provides an average particle size that is at least 67% and no more than 150% the average particle size prior to recovery of the solid composition. Even more preferably, the formulation provides an average particle size that is at least 75% and no more than 133% the average particle size prior to recovery of the solid composition.

The second procedure is known as a filter potency test. In this test the concentration of drug after passing the suspension of the nanoparticles through a filter is determined. The solid composition is added to an aqueous solution as described above. The concentration of drug in the so-formed suspension is then determined using standard techniques, such as by high-performance liquid chromatography (HPLC). Next, the suspension is filtered through a filter, and the concentration of drug in the filtered sample is determined via standard techniques. A loss in potency after filtering a sample through a filter is an indication that the nanoparticles in the sample are larger than the filter pore size. Exemplary filters that can be used in this test include a 1-µm glass fiber filter, a 0.45-µm syringe filter, and a 0.2-µm syringe filter. One skilled in the art will understand that the pore size of the filter should be selected to ensure the nanoparticles are not retained on the filter. Generally, the pore size of filter and the range of nanoparticle average diameters are given as follows:

| Filter Pore Size (µm) | Suitable Range of Nanoparticle Diameters (nm) |
|---|---|
| 1 | > 250 |
| 0.45 | 150 to 300 |
| 0.2 | < 200 |

A solid composition is said to provide good resuspendability if the ratio of the concentration of drug in the filtered sample is at least 60% the concentration of drug in the unfiltered sample. Preferably, the concentration of drug in the filtered sample is at least 70% the concentration of drug in the unfiltered sample. Most preferably, the concentration of drug in the filtered sample is at least 80% the concentration of drug in the unfiltered sample.

In an especially preferred embodiment, a composition provides good resuspendability in both of the tests described above.

### Dosage Forms

The compositions of the present invention may be administered using any known dosage form. The nanoparticles may be formulated for administration via oral, subdermal, intranasal, buccal, intrathecal, ocular, intraaural, intraarticular, subcutaneous spaces, vaginal tract, arterial and venous blood vessels, pulmonary tract or intramuscular tissue of an animal, such as a mammal and particularly a human. Oral dosage forms include: powders or granules; tablets; chewable tablets; capsules; unit dose packets, sometimes referred to in the art as "sachets" or "oral powders for constitution" (OPC); syrups; and suspensions. Parenteral dosage forms include reconstitutable powders or suspensions. Topical dosage forms include creams, pastes, suspensions, powders, foams and gels. Ocular dosage forms include suspensions, emulsions, powders, gels, creams, pastes, solid inserts, depots, and implants.

In one embodiment, the compositions of the present invention are capable of improving the concentration of dissolved drug in a use environment relative to a control composition consisting essentially of the drug alone without any of the polymers. In order to determine concentration enhancement *in vitro,* the amount of "free" drug, or solvated drug is measured. By "free" drug is meant drug which is in the form of dissolved drug or present in micelles, but which is not in the nanoparticles or any solid particles larger than 500 nm, such as precipitate. A composition of the invention provides concentration enhancement if, when administered to an aqueous use environment, it provides a free drug concentration that is at least 1.25-fold the free drug concentration provided by the control composition. Preferably, the free drug concentration provided by the compositions of the invention are at least about 1.5-fold, more preferably at least about 2-fold, and most preferably at least about 3-fold that provided by the control composition.

Alternatively, the compositions of the present invention, when dosed orally to a human or other animal, provide an AUC in drug concentration in the blood plasma or serum (or relative bioavailability) that is at least 1.25-fold that observed in comparison to the control composition. Preferably, the blood AUC is at least about 2-fold, more preferably at least about 3-fold, even more preferably at least about 4-fold, still more preferably at least about 6-fold, yet more preferably at least about 10-fold, and most preferably at least about 20-fold that of the control composition. The determination of AUCs is a well-known procedure and is described, for example, in Welling, "Pharmacokinetics Processes and Mathematics," ACS Monograph 185 (1986).

Alternatively, the compositions of the present invention, when dosed orally to a human or other animal, provide a maximum drug concentration in the blood plasma or serum (Cₘₐₓ) that is at least 1.25-fold that observed in comparison to the control composition. Preferably, the Cₘₐₓ is at least about 2-fold, more preferably at least about 3-fold, even more preferably at least about 4-fold, still more preferably at least about 6-fold, yet more preferably at least about 10-fold, and most preferably at least about 20-fold that of the control composition. Thus, compositions that meet the *in vitro* or *in vivo* performance criteria, or both, are considered to be within the scope of the invention.

### Examples

### Drugs Used in Examples

The following drugs were used in the examples as described below.

Drug 1 was 4-(5-methyl-3-phenyl-4-isoxazolyl) benzenesulfonamide, also known as valdecoxib, having the structure:

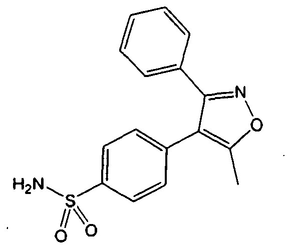

Drug 1 has a solubility in water of about 10 µg/mL, and a Log P value of about 3.0. The T_{g} of non-crystalline Drug 1 was determined by DSC analysis to be 55°C, while the Tₘ of crystalline Drug 1 was 170°C.

Drug 2 was [2R,4S] 4-[acetyl-(3,5-bis-trifluoromethyl-benzyl)-amino]-2-ethyl-6-trifluoromethyl-3,4-dihydro-2H-quinoline-1-carboxylic acid isopropyl ester, having the structure:

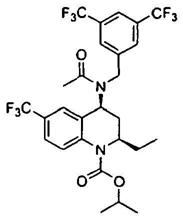

Drug 2 has a solubility in water of less than 1 µg/mL, and a Log P value of 6.7. The T_{g} of non-crystalline Drug 2 was determined by DSC analysis to be 46°C, while the Tₘ of crystalline Drug 2 was 111 °C.

Drug 3 was 4-[5-(4-methylphenyl)-3-(trifluoromethyl)-1H-pyrazol-1-yl] benzenesulfonamide, also known as celecoxib, having the structure:

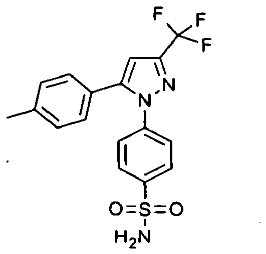

Drug 3 has a solubility in a model fasted duodenal (MFD) solution (phosphate buffered saline [PBS] containing 7.3 mM sodium taurocholic acid and 1.4 mM of 1-palmitoyl-2-oleyl-sn-glycero-3-phosphocholine) of about 40 µg/mL, and a LogP value of 3.75. The T_{g} of non-crystalline Drug 3 was determined by DSC analysis to be 54°C, while the Tₘ of crystalline Drug 3 was 158°C.

### Polymers Used in Examples

The following poorly aqueous soluble non-ionizable polymers were used in the examples: ethylcellulose (ETHOCEL® Viscosity 4, Dow Chemical Co., Midland, MI); cellulose acetate (CA 398-10, available from Eastman Chemical Co., Kingsport, TN); cellulose acetate butyrate (CAB-551-0.01, available from Eastman Chem. Co.).

These polymers were evaluated using the following procedure to verify that they are poorly aqueous soluble. First, 0.2 mg/mL of the polymer was added to a PBS solution consisting of 20 mM Na₂HPO₄ 47 mM KH₂PO₄, 87 mM NaCl, and 0.2 mM KCI, adjusted to pH 6.5 with NaOH. The polymer was stirred in the PBS solution for approximately 1 hour at room temperature. Next, the polymer solution was filtered through a nylon 0.45 µm filter that was weighed dry prior to filtration. The filter was dried overnight at 40°C, and weighed the following morning. The amount of soluble polymer was calculated from the amount of polymer added to form the polymer solution minus the amount of polymer remaining on the filter. The results of these tests are shown in Table 1 and show that all of the polymers tested are poorly aqueous soluble.

**Table 1**

| Example Polymer Example | Amount Soluble at pH 6.5 (mg/mL) | Observations |
|---|---|---|
| Ethylcellulose | <0.001 | Fine particle suspension |
| Cellulose Acetate | 0.03 | Fine particle suspension |
| Cellulose Acetate Butyrate | 0.01 | Fine particle suspension |

The following anionic cellulosic polymers were used in the examples: AQOAT-L (hydroxypropyl methylcellulose acetate succinate, HPMCAS-L) manufactured by Shin Etsu (Tokyo, Japan), carboxymethyl cellulose acetate butyrate (CMCAB-641-0.5) available from Eastman Chemical Company (Kingsport, Tennessee), cellulose acetate phthalate (CAP, available from Eastman Chemical Co.), and ethylcellulose succinate (ECS).

The ECS was synthesized as follows. First, 200 mL of glacial acetic acid was added to a round bottom flask equipped with a water condenser and a stir bar and placed into an oil bath set at 85°C. The flask was purged with argon. To this, 20.011 g of ethylcellulose (ETHOCEL, Viscosity 4) and 20.028 g of sodium acetate were added and allowed to dissolve. Once complete dissolution of the ethylcellulose occurred, 15.001 g of succinic anhydride was added and the mixture was allowed to react overnight. The reaction mixture was quenched into about 1600 mL of water, and the suspension was blended in a blender. The polymer was filtered using a Buchner funnel, then blended and precipitated again in about 1600 mL water. The polymer was filtered and precipitated a third time in about 1600 mL hot water. The precipitate was filtered and dried *in vacuo* to yield an off-white solid.

### Example 1

Nanoparticles containing Drug 1 were prepared using ethylcellulose and ECS using the following procedure. First, 20 mg Drug 1, 30 mg of the poorly aqueous soluble non-ionizable polymer ethylcellulose (ETHOCEL viscosity 4), and 30 mg of the anionic cellulosic polymer ECS were dissolved in 4 mL ethyl acetate to form an organic solution. This organic solution was then poured into 20 mL of deionized water and emulsified for 4 min using a Kinematica Polytron 3100 rotor/stator (Kinematica AG, Lucerne, Switzerland) at 10,000 rpm. The solution was further emulsified using a microfluidizer (Microfluidics [Newton, MA] model M-110L F12Y with Z chamber), with an inlet pressure of 80 psi for 4 minutes. The ethyl acetate was then removed from the emulsion using a rotary evaporator (28°C, 200 rpm, 20 min), resulting in an aqueous suspension of nanoparticles. The nanoparticles of Example 1 contained 25:37.5:37.5 Drug 1:ethylcellulose:ECS.

### Light Scattering Analysis

The particle size of the nanoparticles in the aqueous suspension was determined using dynamic light scattering (DLS) as follows. First, the aqueous suspension was filtered using a 1 µm glass membrane filter (Anatop filter, Whatman), and poured into a cuvette. Dynamic light-scattering was measured using a Brookhaven Instruments (Holtsville, NY) BI-200SM particle size analyzer with a BI-9000AT correlator. The sums of exponentials from the autocorrelation functions were analyzed using CONTIN software to extract size distributions from the samples. The cumulant diameter of the nanoparticles of Example 1 was found to be 211 nm, with a polydispersity of 0.20. After storage for 24 hours at ambient conditions the cumulant diameter of the nanoparticles was 219 nm with a polydispersity of 0.20. This demonstrates the nanoparticles of Example 1 did not agglomerate.

### Control 1

As a control, nanoparticles were prepared containing Drug 1 and the poorly aqueous soluble non-ionizable polymer ethylcellulose, but without an anionic cellulosic polymer. To form Control 1 nanoparticles, 40.6 mg Drug 1 and 123.8 mg ethylcellulose were dissolved in 5 mL methylene chloride to form an organic solution. The organic solution was then poured into 20 mL deionized water, and emulsified as described in Example 1. The methylene chloride was removed from the emulsion using a rotary evaporator, resulting in an aqueous suspension of nanoparticles. The aqueous suspension was analyzed using DLS as described in Example 1. The cumulant diameter of the nanoparticles was 815 nm, with a polydispersity of 0.41. Visual observation of the aqueous suspension confirmed large agglomerated and stringy material. These results show that nanoparticles made using an emulsion method without an anionic cellulosic polymer do not provide a stable formulation.

### Example 2

Nanoparticles containing Drug 3 were prepared using ethylcellulose and the anionic cellulosic polymer, hydroxypropyl methylcellulose acetate succinate, HPMCAS-L, (AQOAT-L, available from Shin Etsu). The organic solution consisted of 80 mg Drug 3, 160 mg ethylcellulose, and 80 mg HPMCAS-L dissolved in 4 mL ethyl acetate. The organic solution was then poured into 20 mL deionized water and emulsified for 3 min using a Kinematica Polytron 3100 rotor/stator at 10,000 rpm. The solution was further emulsified using a microfluidizer for 6 minutes. The ethyl acetate was removed from the emulsion using a rotary evaporator (30°C, 160 rpm, 7 min), resulting in an aqueous suspension of nanoparticles.

### Light Scattering analysis

The aqueous suspension of Example 2 was filtered using a 1 µm filter (Acrodisk®, Pall Co., Ann Arbor, MI), diluted 1:15, and analyzed using DLS. The cumulant diameter of the nanoparticles in aqueous suspension was 149 nm with a polydispersity of 0.22.

The aqueous suspension was allowed to stand unmixed for 4 days (ambient conditions) to measure stability. Each day, the suspension was analyzed using DLS analysis. The results, shown in Table 2, show that particle size is maintained in the suspension during storage, indicating that the nanoparticles of Example 2 remain small and unagglomerated.

**Table 2**

| Sample | Particle size (nm) |
|---|---|
| Initial | 149 |
| 2 days | 151 |
| 3 days | 146 |
| 4 days | 152 |

### Example 3

Nanoparticles of Drug 3 were made containing ethylcellulose and HPMCAS-L using the following procedure. First, 75 mg Drug 3 and 75 mg ethylcellulose were dissolved in 5 mL ethyl acetate to form an organic solution. Next, 100 mg of HPMCAS-L was dissolved in 20 mL of water adjusted to pH 7.7 using 1 M NaOH to form an aqueous solution. The 5 mL organic solution was then added to 10 mL of the aqueous solution and the mixture was emulsified for 3 min using a Kinematica Polytron 3100 rotor/stator at 10,000 rpm. The solution was further emulsified using a microfluidizer for 6 minutes. The ethyl acetate was removed from the emulsion using a rotary evaporator (30°C, 160 rpm, 7 min), resulting in an aqueous suspension of nanoparticles consisting of 37.5:37.5:25 Drug 3:ethylcellulose: HPMCAS-L.

### Light Scattering Analysis

The aqueous suspension of Example 3 was analyzed using DLS as described in Example 1. The cumulant diameter of the nanoparticles in aqueous suspension was 165 nm.

### Examples 4-5

Nanoparticles of Drug 3 were made containing ethylcellulose and carboxymethyl cellulose acetate butyrate (CMCAB-641-0.5 available from Eastman Chemical Company, Kingsport, Tennessee) using a precipitation method as follows. For Example 4, 40 mg Drug 3, 50 mg ethylcellulose, and 10 mg CMCAB were added to 10 mL of acetone and sonicated for 15 minutes to dissolve the materials. The resulting solution was then filtered through a 1-µm Whatman filter to form the organic solution. A 1-mL sample of the organic solution was then rapidly added to 9 mL of distilled water, resulting in the formation of nanoparticles in suspension.

The nanoparticles of Example 5 were made using the same procedure except that the organic solution consisted of 40 mg Drug 3, 40 mg ethylcellulose, and 20 mg CMCAB dissolved in acetone.

### Light Scattering Analysis

The nanoparticle suspension of Examples 4 and 5 were analyzed by DLS using the procedures outlined in Example 1. The cumulant diameter of the Example 4 nanoparticles in aqueous suspension was 97 nm with a polydispersity of 0.30. The cumulant diameter of the Example 5 nanoparticles in aqueous suspension was 101 nm with a polydispersity of 0.28.

### Example 6

Nanoparticles containing Drug 2, cellulose acetate (CA 398-10, available from Eastman Chemical Co., Kingsport, TN), and the anionic cellulosic polymer cellulose acetate phthalate (CAP, available from Eastman Chemical Co.) were prepared using a precipitation process. First, a water-miscible organic solution was formed by mixing 25.3 mg Drug 2, 38.0 mg CA, and 37.6 mg CAP in 10 mL acetone to form a clear solution. The aqueous solution consisted of 90 mL deionized water. To form the nanoparticles, a pipette containing the organic solution was inserted under the surface of the aqueous solution, and delivered into the stirring vortex all at once, rapidly precipitating the nanoparticles. The acetone was removed using a rotary evaporator (30°C, 150 rpm, 10 min), resulting in an aqueous suspension of nanoparticles.

The aqueous suspension of Example 6 was analyzed using DLS. The average diameter of the nanoparticles was 193 nm with a polydispersity of 0.14.

The aqueous suspension was allowed to stand unmixed (ambient conditions) to measure stability. After about 24 hours, the suspension was analyzed again using DLS, and the average diameter of the nanoparticles was 143, with a polydispersity of 0.30. The results indicate that the nanoparticles of Example 6 remain unagglomerated in suspension.

### Isolation of Solid Nanoparticles

To form a solid composition, 100 mg of trehalose was added to the aqueous suspension of Example 6. The resulting solution was frozen rapidly using liquid nitrogen, then lyophilized overnight to obtain a dry powder.

### PXRD Evaluation

The solid composition of Example 6 was examined using powder x-ray diffraction with a Bruker AXS D8 Advance diffractometer to determine the crystalline character of the drug in the nanoparticles. The sample was packed in Lucite a sample cup fitted with Si(511) plates as the bottom of the cup to give no background signal. The sample was spun in the ϕ plane at a rate of 30 rpm to minimize crystal orientation effects. The x-ray source (KCu_{α}, λ = 1.54 A) was operated at a voltage of 45 kV and a current of 40 mA. Data were collected in continuous detector scan mode at a scan speed of 8 seconds/step and a step size of 0.02°/step. Diffractograms were collected over the 2θ range of 4° to 40°. The results are shown in FIG 1. The nanoparticles of Example 6 exhibited a diffraction pattern showing an amorphous halo, and no sharp peaks characteristic of crystalline drug. These data indicate that the drug in the nanoparticles of Example 6 is non-crystalline.

### Example 7

Nanoparticles containing Drug 2, cellulose acetate butyrate (CAB-551-0.01, available from Eastman Chemical Co.), and the anionic cellulosic polymer carboxymethyl cellulose acetate butyrate (CMCAB-641-0.5, available from Eastman Chemical Co.), were prepared using a precipitation process as follows. First, a water-miscible organic solution was formed by mixing 25.0 mg Drug 2, 37.8 mg CAB, and 37.5 mg CMCAB in 10 mL acetone to form a clear solution. The aqueous solution consisted of deionized water. To form the nanoparticles, a pipette containing 1 mL of the organic solution was inserted under the surface of 9 mL aqueous solution, and delivered into the stirring vortex all at once, rapidly precipitating the nanoparticles. The acetone was removed using a rotary evaporator (30°C, 150 rpm, 5 min), resulting in an aqueous suspension of nanoparticles.

The aqueous suspension of Example 7 was analyzed using DLS. The average diameter of the nanoparticles was 101 nm with a polydispersity of 0.26.

The aqueous suspension was allowed to stand unmixed (ambient conditions) to measure stability. After about 24 hours, the suspension was analyzed again using DLS, and the average diameter of the nanoparticles was 143, with a polydispersity of 0.27.

## Claims

1. A pharmaceutical composition comprising nanoparticles, said nanoparticles comprising:
(a) a drug having a solubility in water of less than 5 mg/mL over the pH range of 6.5 to 7.5 at 25°C, at least 90 wt% of said drug being non-crystalline;
(b) a non-ionizable polymer having a solubility of less than 0,1 mg/mL at a concentration of 0,2 mg/mL in a phosphate buffered aqueous saline solution at pH 6.5;
and
(c) an anionic cellulosic polymer;
wherein said nanoparticles have an average size of less than 500 nm and said drug, said non-ionizable polymer, and said anionic cellulosic polymer collectively constitute at least 80 wt% of said nanoparticles.

2. The composition of claim 1 wherein said nanoparticles have the following composition: from 5 wt% to 75 wt% said drug, from
10 wt% to 75 wt% said non-ionizable polymer, and from 5 wt% to 60 wt% said anionic cellulosic polymer.

3. The composition of any of the preceding claims wherein said non-ionizable polymer is selected from the group consisting of methylcellulose, ethylcellulose, propylcellulose, butylcellulose, cellulose acetate, cellulose propionate, cellulose butyrate, cellulose acetate butyrate, cellulose acetate propionate, methyl cellulose acetate, methyl cellulose propionate, methyl cellulose butyrate, ethyl cellulose acetate, ethyl cellulose propionate, ethyl cellulose butyrate, low-substituted hydroxypropyl cellulose, hydroxypropyl methylcellulose acetate, hydroxypropyl methylcellulose propionate, hydroxypropyl methylcellulose butyrate, poly(lactide), poly(glycolide), poly(ε-caprolactone), poly(lactide-co-glycolide), poly(lactide-co-ε-caprolactone), poly(ethylene oxide-co-ε-caprolactone), poly(ethylene oxide-co-lactide), poly(ethylene oxide-co-lactide-co-glycolide), poly(isobutyl)cyanoacrylate, and poly(hexyl)cyanoacrylate,

4. The composition of any of the preceding claims wherein said non-ionizable polymer is selected from the group consisting of ethylcellulose, cellulose acetate, cellulose propionate, cellulose butyrate, and cellulose acetate butyrate.

5. The composition of any of the preceding claims wherein said anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, hydroxypropyl methyl cellulose acetate phthalate, cellulose acetate trimellitate, hydroxypropyl methyl cellulose acetate trimellitate, ethyl cellulose succinate, ethyl cellulose phthalate, ethyl cellulose trimellitate, cellulose acetate succinate, and methylcellulose acetate succinate.

6. The composition of any of the preceding claims wherein said non-ionizable polymer is selected from the group consisting of ethylcellulose, cellulose acetate, cellulose propionate, cellulose butyrate, and cellulose acetate butyrate, and said anionic cellulosic polymer is selected from the group consisting of hydroxypropyl methyl cellulose acetate succinate, hydroxypropyl methyl cellulose phthalate, carboxymethyl ethylcellulose, carboxymethylcellulose acetate butyrate, cellulose acetate phthalate, cellulose acetate trimellitate, ethyl cellulose succinate, and cellulose acetate succinate.

7. The composition of any of the preceding claims wherein said non-ionizable polymer is selected from the group consisting of ethylcellulose, cellulose acetate, and cellulose acetate butyrate, and said anionic cellulosic polymer is selected from the group consisting of hydroxypropyl methyl cellulose acetate succinate, carboxymethyl ethylcellulose, carboxymethylcellulose acetate butyrate, cellulose acetate phthalate, and ethyl cellulose succinate.

8. The composition of any of the preceding claims further comprising water, wherein said nanoparticles are suspended in said water.

9. A solid composition comprising (a) the nanoparticles of any of the preceding claims and (b) a matrix material.

## Patentansprüche

1. Pharmazeutische Zusammensetzung, die Nanopartikel umfasst, wobei die Nanopartikel umfassen:
(a) ein Arzneimittel mit einer Löslichkeit in Wasser von weniger als 5 mg/ml über den pH-Bereich von 6,5 bis 7,5 bei 25°C, wobei 90 Gewichtsprozent des Arzneimittels nichtkristallin sind;
(b) ein nichtionisierbares Polymer mit einer Löslichkeit von weniger als 0,1 mg/ml bei einer Konzentration von 0,2 mg/ml in einer Phosphat-gepufferten wässrigen Kochsalzlösung mit pH 6,5 und
(c) ein anionisches Cellulosepolymer;
wobei die Nanopartikel eine durchschnittliche Größe von weniger als 500 nm haben, und das Arzneimittel, das nichtionisierbare Polymer und das anionische Cellulosepolymer zusammen wenigstens 80 Gewichtsprozent der Nanopartikel bilden.

2. Zusammensetzung gemäß Anspruch 1, wobei die Nanopartikel die folgende Zusammensetzung haben: 5 Gewichtsprozent bis 75 Gewichtsprozent des Arzneimittels, 10 Gewichtsprozent bis 75 Gewichtsprozent des nichtionisierbaren Polymers und 5 Gewichtsprozent bis 60 Gewichtsprozent des anionischen Cellulosepolymers.

3. Zusammensetzung gemäß einem der vorangehenden Ansprüche, wobei das nichtionisierbare Polymer ausgewählt ist aus der Gruppe, bestehend aus Methylcellulose, Ethylcellulose, Propylcellulose, Butylcellulose, Celluloseacetat, Cellulosepropionat, Cellulosebutyrat, Celluloseacetatbutyrat, Celluloseacetatpropionat, Methylcelluloseacetat, Methylcellulosepropionat, Methylcellulosebutyrat, Ethylcelluloseacetat, Ethylcellulosepropionat, Ethylcellulosebutyrat, niedrigsubstituierter Hydroxypropylcellulose, Hydroxypropylmethylcelluloseacetat, Hydroxypropylmethylcellulosepropionat, Hydroxypropylmethylcellulosebutyrat, Poly(lactid), Poly(glycolid), Poly(ε-caprolacton), Poly(lactid-co-glycolid), Poly(lactid-co-ε-caprolacton), Poly(ethylenoxid-co-ε-caprolacton), Poly(ethylenoxid-co-lactid), Poly(ethylenoxid-co-lactid-co-glycolid), Poly(isobutyl)cyanoacrylat und Poly(hexyl)cyanoacrylat.

4. Zusammensetzung gemäß einem der vorangehenden Ansprüche, wobei das nichtionisierbare Polymer aus der Gruppe, bestehend aus Ethylcellulose, Celluloseacetat, Cellulosepropionat, Cellulosebutyrat und Celluloseacetatbutyrat, ausgewählt ist.

5. Zusammensetzung gemäß einem der vorangehenden Ansprüche, wobei das anionische Cellulosepolymer aus der Gruppe, bestehend aus Hydroxypropylmethylcelluloseacetatsuccinat, Hydroxypropylmethylcellulosephthalat, Carboxymethylethylcellulose, Carboxymethylcelluloseacetatbutyrat, Celluloseacetatphthalat, Hydroxypropylmethylcelluloseacetatphthalat, Celluloseacetattrimellitat, Hydroxypropylmethylcelluloseacetattrimellitat, Ethylcellulosesuccinat, Ethylcellulosephthalat, Ethylcellulosetrimellitat, Celluloseacetatsuccinat und Methylcelluloseacetatsuccinat, ausgewählt ist.

6. Zusammensetzung gemäß einem der vorangehenden Ansprüche, wobei das nichtionisierbare Polymer aus der Gruppe, bestehend aus Ethylcellulose, Celluloseacetat, Cellulosepropionat, Cellulosebutyrat und Celluloseacetatbutyrat, ausgewählt ist und das anionische Cellulosepolymer aus der Gruppe, bestehend aus Hydroxypropylmethylcelluloseacetatsuccinat, Hydroxypropylmethylcellulosephthalat, Carboxymethylethylcellulose, Carboxymethylcelluloseacetatbutyrat, Celluloseacetatphthalat, Celluloseacetattrimellitat, Ethylcellulosesuccinat und Celluloseacetatsuccinat, ausgewählt ist.

7. Zusammensetzung gemäß einem der vorangehenden Ansprüche, wobei das nichtionisierbare Polymer aus der Gruppe, bestehend aus Ethylcellulose, Celluloseacetat und Celluloseacetatbutyrat, ausgewählt ist und das anionische Cellulosepolymer aus der Gruppe, bestehend aus Hydroxypropylmethylcelluloseacetatsuccinat, Carboxymethylethylcellulose, Carboxymethylethylcelluloseacetatbutyrat, Celluloseacetatphthalat und Ethylcellulosesuccinat, ausgewählt ist.

8. Zusammensetzung gemäß einem der vorangehenden Ansprüche, die außerdem Wasser umfasst, wobei die Nanopartikel in dem Wasser suspendiert sind.

9. Feste Zusammensetzung, umfassend (a) die Nanopartikel gemäß einem der vorangehenden Ansprüche und (b) ein Matrixmaterial.

## Revendications

1. Composition pharmaceutique comprenant des nanoparticules, lesdites nanoparticules comprenant :
(a) un médicament ayant une solubilité dans l'eau inférieure à 5 mg/mL sur la plage de pH allant de 6,5 à 7,5 à 25 °C, au moins 90 % en poids dudit médicament étant non cristallin ;
(b) un polymère non ionisable ayant une solubilité inférieure à 0,1 mg/mL à une concentration de 0,2 mg/mL dans une solution saline aqueuse de tampon phosphate à pH 6,5 ;
et
(c) un polymère cellulosique anionique ;
dans laquelle lesdites nanoparticules ont une taille moyenne inférieure à 500 nm et ledit médicament, ledit polymère non ionisable, et ledit polymère cellulosique anionique constituent collectivement au moins 80 % en poids desdites nanoparticules.

2. Composition selon la revendication 1, dans laquelle lesdites nanoparticules ont la composition suivante :de 5 % en poids à 75 % en poids dudit médicament, de 10 % en poids à 75 % en poids dudit polymère non ionisable, et de 5 % en poids à 60 % en poids dudit polymère cellulosique anionique.

3. Composition selon l'une quelconque des revendications précédentes, dans laquelle ledit polymère non ionisable est choisi dans le groupe comprenant la méthylcellulose, l'éthylcellulose, la propylcellulose, la butylcellulose, l'acétate de cellulose, le propionate de cellulose, le butyrate de cellulose, l'acétate butyrate de cellulose, l'acétate propionate de cellulose, l'acétate de méthylcellulose, le propionate de méthylcellulose, le butyrate de méthylcellulose, l'acétate d'éthylcellulose, le propionate d'éthylcellulose, le butyrate d'éthylcellulose, l'hydroxypropyl cellulose substituée par un groupement de faible poids moléculaire, l'acétate d'hydroxypropyl méthylcellulose, le propionate d'hydroxypropyl méthylcellulose, le butyrate d'hydroxypropyl méthylcellulose, le poly(lactide), le poly(glycolide), la poly(ε-caprolactone), le poly(lactide-co-glycolide), le poly(lactide-co-ε-caprolactone), le poly(oxyde d'éthylène-co-ε-caprolactone), le poly(oxyde d'éthylène-co-lactide), le poly(oxyde d'éthylène-co-lactide-co-glycolide), le poly(isobutyl)cyanoacrylate, et le poly(hexyl)cyanoacrylate.

4. Composition selon l'une quelconque des revendications précédentes, dans laquelle ledit polymère non ionisable est choisi dans le groupe comprenant l'éthylcellulose, l'acétate de cellulose, le propionate de cellulose, le butyrate de cellulose, et l'acétate butyrate de cellulose.

5. Composition selon l'une quelconque des revendications précédentes, dans laquelle ledit polymère cellulosique anionique est choisi dans le groupe comprenant l'acétate succinate d'hydroxypropyl méthylcellulose, le phtalate d'hydroxypropyl méthylcellulose, la carboxyméthyl éthylcellulose, l'acétate butyrate de carboxyméthylcellulose, l'acétate phtalate de cellulose, l'acétate phtalate d'hydroxypropyl méthylcellulose, l'acétate trimellitate de cellulose, l'acétate trimellitate d'hydroxypropyl méthylcellulose, le succinate d'éthylcellulose, le phtalate d'éthylcellulose, le trimellitate d'éthylcellulose, l'acétate succinate de cellulose, et l'acétate succinate de méthylcellulose.

6. Composition selon l'une quelconque des revendications précédentes, dans laquelle ledit polymère non ionisable est choisi dans le groupe comprenant l'éthylcellulose, l'acétate de cellulose, le propionate de cellulose, le butyrate de cellulose, et l'acétate butyrate de cellulose, et dans laquelle ledit polymère cellulosique anionique est choisi dans le groupe comprenant l'acétate succinate d'hydroxypropyl méthylcellulose, le phtalate d'hydroxypropyl méthylcellulose, la carboxyméthyl éthylcellulose, l'acétate butyrate de carboxyméthylcellulose, l'acétate phtalate de cellulose, l'acétate trimellitate de cellulose, le succinate d'éthylcellulose, et l'acétate succinate de cellulose.

7. Composition selon l'une quelconque des revendications précédentes, dans laquelle ledit polymère non ionisable est choisi dans le groupe comprenant l'éthylcellulose, l'acétate de cellulose, et l'acétate butyrate de cellulose, et dans laquelle ledit polymère cellulosique anionique est choisi dans le groupe comprenant l'acétate succinate d'hydroxypropyl méthylcellulose, la carboxyméthyl éthylcellulose, l'acétate butyrate de carboxyméthylcellulose, l'acétate phtalate de cellulose, et le succinate d'éthylcellulose.

8. Composition selon l'une quelconque des revendications précédentes, comprenant en outre de l'eau, dans laquelle lesdites nanoparticules sont en suspension dans ladite eau.

9. Composition solide comprenant (a) les nanoparticules selon l'une quelconque des revendications précédentes et (b) un matériau matriciel.
